# EUROPEAN PATENT APPLICATION

(11) **EP 1 282 063 A2**
(43) Date of publication of application: **05.02.2003**
(21) Application number: 02255355.6
(22) Date of filing: 31.07.2002
(51) Int. Cl.: G06F 19/00

(54) **Computer-aided diagnosis system**

(30) Priority: 03.08.2001 JP 2001236175
(71) Applicant: KONICA CORPORATION, Tokyo (JP)
(72) Inventor: Kasai, Satoshi, c/o Konica Corporation, Hino-shi, Tokyo 191-8511 (JP)
(74) Representative: Rees, Alexander Ellison

(57) **Abstract**

A computer-aided diagnosis system comprising: a computer-aided diagnosis software server having a computer-aided diagnosis software capable of processing a radiographic image data to detecting an abnormality; and an image determining apparatus interconnected with the software server via a network, wherein the image determining apparatus determines the radiographic image data to be processed, downloads the computer-aided diagnosis software from the software server, and processes the radiographic image data by utilizing the downloaded computer-aided diagnosis software.

## Description

### FIELD OF THE INVENTION

The present invention relates to a computer-aided diagnosis system.

### BACKGROUND OF THE INVENTION

With objects for lightening a burden of a doctor who reads radiographs and for reducing oversights of abnormal shadows, there has been developed a computer-aided diagnosis (hereinafter referred to as CAD) that detects abnormal shadows such as a lung cancer and a breast cancer. In the case of the breast cancer, there has been reported widely the detection of mass shadow and micro-calcification which are called two great views.

### Papers relating to the detection of two great views

### 1) Mass shadow

· Method to detect by comparing a left breast with a right breast (Med. Phys>, Vol. 21. No. 3, pp. 445 - 452)
· Method to detect by using Iris Filter (shingakuron (D-II), Vol. J75-D-11, No. 3, pp. 663-670, 1992)
· Method to detect by using an iris filter (shingakuron (D-II), Vol. J75-D-11, No. 3, pp. 663-670, 1992)
· Method to detect by using Quoit filter (shingakuron (D-II), Vol. J76-D-11, No. 3, pp. 279-287, 1993)
· Method to detect by binary-encoding based on a histogram of a pixel value in a divided breast region (JAMIT Frontier Collection of papers, pp. 84-85, 1995)
· Microscopic deflection difference filter that takes minimum output of many Laplacian filters having polarity (shingakuron (D-II), Vol. J76-D-11, No. 2, pp. 241-249, 1993)

### 2) Micro-calcification cluster

· Method to localize a doubtful area for calcification from a breast area and to eliminate a false positive data based on a standard deviation value of optical density difference and boundary density difference of calcifications (IEEE Trans Biomed Eng BME-26 (4): 213-219, 1979)
· Method to detect by using an image subjected to Laplacian filter processing (shingakuron (D-II), Vol. J71-D-11, No. 10, pp. 1994-20016, 1988)
· Method to detect by using an image subjected to morphology analysis for inhibiting an influence of background pattern such as mammary gland (shingakuron (D-II), Vol. J71-D-11, No. 7, pp. 1170-1176, 1992)

Further, there has also been developed a computer-aided diagnosis which is conducted by preparing a subtraction image from a previous image, by using chest postero-anterior images. Such process is known as a temporal subtraction processing.

The typical temporal subtraction processing is explained in the following papers.
Kano A, Doi K, MacMahon H et al.
"Digital image subtraction of temporally sequential chest images for detection of interval change" Med Phys 21 (3), 453-461, 1994

In the past, each facility using a CAD system has had its own CAD system. In this case, it has sometimes happened that each facility had its different version and maintenance was troublesome. Further, in the case of occurrence of bugs, all facilities had to take actions. In addition, it has been necessary to purchase an exclusive equipment which was expensive.

### SUMMARY OF THE INVENTION

The invention has been achieved in view of the problems stated above, and an object of the invention is to provide a computer-aided diagnosis system wherein each CAD system is connected with others through a network and efficiency of utilization is improved.

### Structure 1.

A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed, downloads the computer-aided diagnosis software from the software server, and processes the radiographic image data by utilizing the downloaded computer-aided diagnosis software.

In the aforesaid structure, it is possible to administrate CAD algorithm easily, by connecting an image determining apparatus of facilities representing clients with a computer-aided diagnosis software server (hereinafter referred to as CAD server) through a network. It is further possible to reduce an amount of communication on the network and to shorten a period of time required to obtain results, by downloading the CAD software (hereinafter referred as CAD software) without sending images to CAD server.

### Structure 2

The computer-aided diagnosis system of Structure 1, wherein the software server has a plurality of different kinds of computer-aided diagnosis softwares from each other, and the image determining apparatus selects the computer-aided diagnosis software to process the radiographic image data and uploads information of the result of selection to the software server.

In the structure mentioned above, when the CAD server has a plurality of CAD softwares, it is possible to make the image determining apparatus to determine the computer-aided diagnosis software to be downloaded.

### Structure 3

The computer-aided diagnosis system of Structure 1, wherein the image determining apparatus comprises:
a storing device capable of storing at least one of the radiographic image data, processed result data of processing the radiographic image data by the computer-aided diagnosis software, and information relating to the radiographic image data; and
an output device capable of outputting at least one of the radiographic image data, the processed result data and the information relating to the radiographic image data.

In the aforesaid structure, it is possible to call the images and the results of the detection by the computer-aided diagnosis software again and to call the necessary information in case of need to confirm it, by providing a storing device for preserving images, results of the detection by the computer-aided diagnosis software and information relating to images, and an output device for indicating determined images and information stored.

### Structure 4

The computer-aided diagnosis system of Structure 3, wherein the information relating to the radiographic image data is at least one of patient information and an exposure condition of the radiographic image data.

In the aforesaid structure, when patient information, radiographing conditions and results of the computer-aided diagnosis software are outputted to an output apparatus together with images, the images may be correlated with a patient and may be of value for reference, thus oversights and errors can be reduced.

### Structure 5

The computer-aided diagnosis system of Structure 1, wherein the software server charges by at least one of the number of times for the image determining apparatus to download the computer-aided diagnosis software, the kind of downloaded computer-aided diagnosis software and the connecting time from the image determining apparatus to the software server.

In the aforesaid structure, it is possible to control a fee for the single use of CAD software and thereby to create a system which can be used easily even by a user whose frequency of the use is low, by making only the necessary software among plural CAD software to be available, and by charging fees based on the frequency of the use.

### Structure 6

The computer-aided diagnosis system of Structure 1, wherein the image determining apparatus further comprises a parameter changing device to change the parameter of the computer-aided diagnosis software downloaded from the software server.

In the aforesaid structure, it is possible to improve capacity of CAD with which CAD is performed with a parameter of CAD complying the radiographing conditions of each facility.

### Structure 7

The computer-aided diagnosis system of Structure 1, wherein a plurality of the image determining apparatus connects to the software server.

### Structure 8

A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed and uploads the determined radiographic image data to the software server, the software server processes the uploaded radiographic image data by utilizing the computer-aided diagnosis software, and the image determining apparatus downloads the processed result data of processing the radiographic image data by the computer-aided diagnosis software from the software server.

In the aforesaid structure, it is possible to administrate CAD algorithm easily, by connecting image determining apparatus of a facility representing client with a CAD server through a network. Further, by sending images to CAD server, it is possible to administrate images processed by CAD software and to administrate CAD results on a unitary basis, and it is also possible to use for another object to compare with previous images.

### Structure 9

The computer-aided diagnosis system of Structure 8, wherein the software server has a plurality of different kinds of computer-aided diagnosis softwares from each other, and the image determining apparatus selects the computer-aided diagnosis software to process the radiographic image data and uploads information of the result of selection to the software server.

In the aforesaid structure, it is possible to make the image determining apparatus to determine the computer-aided diagnosis software to be used for processing.

### Structure 10

The computer-aided diagnosis system of Structure 8, wherein the image determining apparatus comprises:
a storing apparatus capable of storing at least one of the radiographic image data, processed result data of processing the radiographic image data by the computer-aided diagnosis software, and information relating to the radiographic image data; and
an output apparatus capable of outputting at least one of the radiographic image data, the processed result data and the information relating to the radiographic image data.

In the aforesaid structure, it is possible to call the images and the results of the detection by the computer-aided diagnosis software again and to call the necessary information in case of need to confirm it, by providing a storing device for preserving images, results of the detection by the computer-aided diagnosis software and information relating to images, and an output device for indicating determined images and information preserved.

### Structure 11

The computer-aided diagnosis system of Structure 10, wherein the information relating to the radiographic image data is at least one of a patient information and an exposure condition of the radiographic image data.

In the aforesaid structure, when patient information, radiographing conditions and results of the computer-aided diagnosis software are outputted to an output apparatus together with images, the images may be made to correspond to a patient and may be of value for reference, thus oversights and errors can be reduced.

### Structure 12

The computer-aided diagnosis system of Structure 8, wherein the image determining apparatus uploads information relating to the radiographic image data, which is uploaded to the software server, to the software server.

In the aforesaid structure, it is possible to make administration at CAD server to be easy by storing images and information relating to the images together.

### Structure 13

The computer-aided diagnosis system of Structure 12, wherein the information relating to the radiographic image data is at least one of patient information, information of an exposure condition of the radiographic image data and information relating to the image determining apparatus.

In the aforesaid structure, it is possible to upload either one of patient information, radiographing conditions and information about the image determining apparatus in use.

### Structure 14

The computer-aided diagnosis system of Structure 13, wherein the information relating to the image determining apparatus is information of a facility having the image determining apparatus.

In the aforesaid structure, it is possible to distinguish the facility representing the sending source, when facilities each being different are connected through a network.

### Structure 15

The computer-aided diagnosis system of Structure 12, wherein the software server comprises:
an image storing device capable of storing the uploaded radiographic image data; and
a database making device capable of making a database of at least one of processed result data of processing the radiographic image data by the computer-aided diagnosis software and the information relating to the radiographic image data.

In the aforesaid structure, it is possible to preserve images and to make the results to be database, and thereby to administrate, on a unitary basis, image data and patient information, presence or absence of lesions, types of lesions, and information of radiographing conditions, thus, calling and retrieval for previous images and similar cases are easy, and administration thereof is easy. On the other hand, it is possible to improve the computer-aided diagnosis software by using images and information collected intensively, and improvement of accuracy is expected.

### Structure 16

The computer-aided diagnosis system of Structure 15, wherein the information relating to the radiographic image data is information of facility having the image determining apparatus.

In the aforesaid structure, it is possible to distinguish the facility representing the sending source, in the case of the system wherein facilities each being different are connected through a network.

### Structure 17

The computer-aided diagnosis system of Structure 8, wherein the software server comprises:
a previous-case diagnosis data storing device capable of making a database of at least one of data of a diagnosing result of a previous case and data of a previous treatment and storing the data; and
a retrieving device capable of retrieving the data in the storing device,
wherein the image determining apparatus retrieves the data in the storing device by utilizing the retrieving device and downloads at least one of obtained data as the result of retrieving.

In the aforesaid structure, it is possible to retrieve a similar previous case and to download a result of diagnosis for the previous case and medical examination and treatment to the image determining apparatus, thus, it is possible to support a doctor who reads radiographs, which results in an improvement of diagnosis efficiency.

### Structure 18

The computer-aided diagnosis system of Structure 15, wherein software server further comprises a subtraction processing device capable of retrieving a previous data of uploaded radiographic image data in the image storing device and subjects the previous data and the uploaded radiographic image data to a temporal subtraction processing, and the image determining apparatus download the result of the temporal subtraction processing.

In the aforesaid structure, it is possible to retrieve a previous image in images and to utilize the aging difference processing as computer-aided diagnosis, and thereby to give additional information to a doctor who reads radiographs, which results in an improvement of diagnosis efficiency.

### Structure 19

The computer-aided diagnosis system of Structure 8, wherein the software server charges by at least one of the number of times for the image determining apparatus to upload the radiographic image data to the software server, the number of times of processing the uploaded radiographic image data by the computer-aided diagnosis software, and the connecting time from the image determining apparatus to the software server.

In the aforesaid structure, it is possible to charge a fee based on the number of times of uploading of images, or to restrict usable CAD software to charge based on the number of times of the use, and thereby to control a price for single use of CAD software to create a system which can be used easily even by a user whose frequency of use is low.

### Structure 20

The computer-aided diagnosis system of Structure 8, wherein the image determining apparatus further comprises a parameter data making device to make parameter data capable of changing a parameter of the computer-aided diagnosis software in the software server, and the image determining apparatus uploads the parameter data made by the parameter data making device with the radiographic image data to the software server, and the software server applies the uploaded parameter data to the computer-aided diagnosis software.

In the aforesaid structure, it is possible to apply CAD with a parameter of CAD in accordance with radiographing conditions for each facility, and thereby, it is possible to improve efficiency of CAD.

### Structure 21

The computer-aided diagnosis system of Structure 20, wherein a plurality of image determining apparatuses, which belong to different facilities from each other, connect to the software server, and the software server stores the uploaded parameter data depending on the facilities, and the plurality of image determining apparatuses download the uploaded parameter data.

In the aforesaid structure, it is possible to obtain parameter information of other facilities and thereby to use parameter of the facility that has obtained better CAD results, thus, many facilities can obtain high CAD efficiency.

### Structure 22

The computer-aided diagnosis system of Structure 21, wherein the image determining apparatus uploads information of exposure condition of the radiographic image data to the software server, the software server makes a database of the information of exposure condition of the radiographic image data, and the image determining apparatus downloads the information of exposure condition of the radiographic image data with the parameter data from the software server.

In the aforesaid structure, when downloading the parameter of another facility which studies CAD parameter intensively, if radiographing conditions of that facility are downloaded simultaneously, improvement of CAD efficiency is expected even in the facility where downloading was conducted when the same or similar radiographing conditions are used. Or, it is possible to prevent that CAD efficiency and diagnosis efficiency are lowered, by avoiding application of the same parameter under different radiographing condition.

### Structure 23

The computer-aided diagnosis system of Structure 21, wherein the software server charges by at least one of the facility downloading the parameter data and the number of the facilities downloading the parameter data.

In the aforesaid structure, it is possible to build a system wherein a facility that opens its parameters to the public obtains an income, and it is expected that studying of better CAD parameters may be conducted widely on the user level, which leads to improvement of CAD efficiency on the whole.

### Structure 24

The computer-aided diagnosis system of Structure 8, wherein a plurality of image determining apparatus connect the software server.

### Structure 25

A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed, and
when the image determining apparatus downloads the computer-aided diagnosis software from the software server, the image determining apparatus processes the radiographic image data by utilizing the downloaded computer-aided diagnosis software, and
when the image determining apparatus uploads the determined radiographic image data to the software server, the software server processes the uploaded radiographic image data by utilizing the computer-aided diagnosis software, and the image determining apparatus downloads the processed result data of processing the radiographic image data by the computer-aided diagnosis software from the software server.

In the aforesaid structure, it is possible to administrate images on the computer-aided diagnosis software server, by uploading images and information relating to the images. When it is necessary to apply CAD under different conditions (different CAD software and different CAD parameter) for the images which have been processed on the server, downloading the computer-aided diagnosis software on the image determining apparatus has a merit to process quickly, compared with transmitting a large volume of image data on the network.

### BRIEF DESCRIPTION OF THE DRAWINGS

Fig. 1 is a block diagram showing an example of the embodiment of the invention.
Fig. 2 is a diagram showing the relationship of connection of the image determining apparatus.
Fig. 3 is a flow chart of operations for downloading CAD software.
Fig. 4 is a flow chart of operations for uploading images.

### PREFERRED EMBODIMENT OF THE INVENTION

An embodiment of the invention will be explained in detail as follows, referring to the drawings.

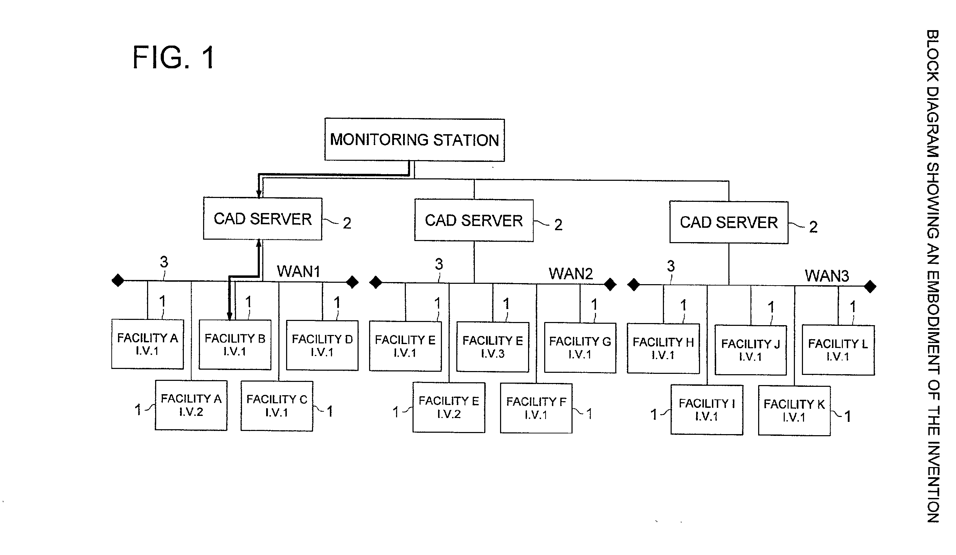

Fig. 1 is a block diagram showing an example of the embodiment of the invention. In Fig. 1, the numeral 1 represents an image determining apparatus (I. V.) that determines digitized radiation images, 2 represents a computer-aided diagnosis software server (hereinafter referred to as CAD server) connected with the image determining apparatus 1 through a network, and 3 represents WAN (wide area network) or LAN (local area network) serving as a network that connects the image determining apparatus 1 with the CAD server 2.

The CAD server 2 houses therein a computer-aided diagnosis software that detects one or plural types of lesion. The image determining apparatus 1 is arranged to conduct processing by using the computer-aided diagnosis software housed in the CAD server 2. In the drawing, CAD servers in quantity corresponding to the number (WAN1 - WAN3) of WANs (or LANs) are used, and three units are shown in this case. To each CAD server 2, there are connected a plurality of image determining apparatuses 1. The image determining apparatus 1 is provided for each facility ranging from one unit to plural units. For example, in the example shown in the drawing, two image determining apparatuses I.V.1 and I.V.2 are connected to facility A.

Between CAD server 2 and image determining apparatus 1, there are conducted downloading of CAD software (CAD server → image determining apparatus), or uploading of images (image determining apparatus → CAD server) and downloading of CAD results (CAD server → image determining apparatus), uploading of information about images (image determining apparatus → CAD server), uploading of CAD parameter for each facility (image determining apparatus → CAD server) and downloading (CAD server → image determining apparatus), and when data base function is provided on CAD server and similar cases can be retrieved, there is conducted downloading (CAD server → image determining apparatus) of diagnosis information of similar images of the retrieved previous cases and diagnoses records.

Information concerning images in this case means information to be added for preserving the uploaded images as data base, and it includes patient information, radiographing conditions, facility information and information relating to the image determining apparatus in use. In the system of the invention, administrating images of plural facilities with CAD server is considered to be basic, and therefore, facility information and information concerning the image determining apparatus in use are especially important. The numeral 4 represents a monitoring station that is connected with these CAD servers 2 and conducts overall administration and control operations. From the monitoring station 4, there is conducted administration of algorithm for CAD server 2.

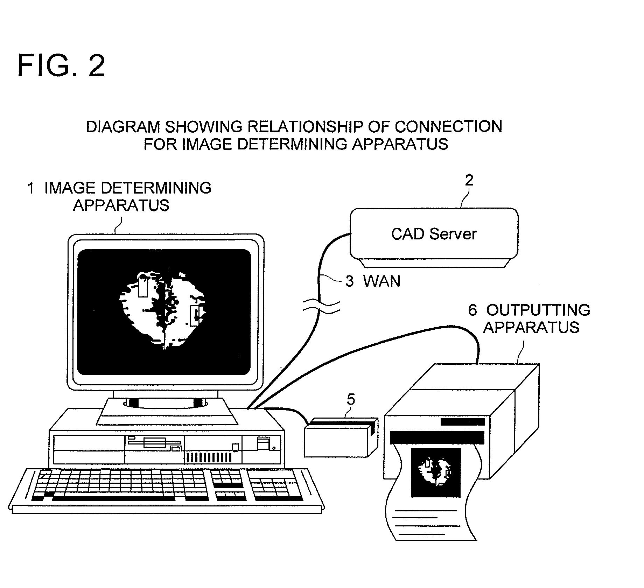

Fig. 2 is a diagram showing the relationship of connection for the image determining apparatus wherein some items which are the same as those in Fig. 1 are given the same symbols. The image determining apparatus 1 is connected with CAD server 2 through WAN 3. As the image determining apparatus 1, there is used practically a personal computer. The numeral 5 represents ID reader which is connected with the image determining apparatus 1 to confirm rights to use customer ID and the image determining apparatus, and the numeral 6 represents an output apparatus for outputting results of CAD, and a printer for outputting on a sheet of paper, for example, and an imager for outputting on a film are used. Operations of the computer-aided diagnosis system structured as in the foregoing will be explained as follows.

### (Overall operations)

The CAD server 2 is installed in each base in Tokyo, Osaka or Nagoya. Each image determining apparatus 1 accesses the CAD server 2 through the network. The image determining apparatus 1 displays an image which is desired to be processed with CAD software by the use of a browser such as an internet explorer or a Netscape Navigator or an exclusive software (hereinafter referred to collectively as viewer).

If entitled after conducting appropriate authentication procedures (log-in with password or ID card) on the viewer, it is possible to conduct log-in. When using an ID card, the ID card (not shown) is made to slide through a groove on ID reader 5 so that a number on the ID card may be read. The ID number thus read is checked whether it agrees with a number registered (stored) in advance or not. If both numbers agree with each other, it means that ID authentication has been completed. Then, desired CAD software is selected to check whether a right to connect to the server and a right to use the selected CAD software are valid or not, and if the right is valid, the CAD software is downloaded from the CAD server 2, and calculation of CAD is carried out on the image determining apparatus 1. On the image determining apparatus 1, results of CAD are indicated together with images, or the results of CAD are outputted on a sheet of paper by output apparatus 6 so that they may be saved as a reference and a record.

### (Operations of CAD server)

CAD server is composed of a browser such as an internet explorer or Netscape Navigator and of an exclusive software. When downloading of AD software from the image determining apparatus is requested, that software is downloaded to comply with the request, when the right is valid, while, when an image to be subjected to CAD is sent from the image determining apparatus, the processing is conducted on the CAD server, and results of the processing are downloaded.

Each CAD server is arranged to be maintained and controlled through remote operations from monitoring station 4, and it is possible to access from a remote location by using lines such as ISDN or an exclusive lines, to provide the latest software to all CAD servers promptly. A software to be administrated by CAD server includes mass shadow detection processing, micro-calcification cluster detection processing, distortion detection processing, mass shadow innocent/malignant discrimination processing, and micro-calcification cluster innocent/malignant discrimination processing, as CAD for mammogram (radiation image of breast), and it includes nodular shadow detection processing, interstitial shadow detection processing and lung cancer detection processing employing CT images, as a chest simple radiograph.

On the CAD server, there may be provided a preserving device that preserves uploaded images and information relating to the images. In this case, it is also possible to conduct administration of images subjected to CAD. Information relating to images means radiographing conditions such as patient information such as a name of a hospital, a patient name and patient ID and ages, radiographing conditions such as voltage of X-ray tube, mAs (milliampere second) value, radiographing direction, a type of a filter, a type of a tube, a radiographing date and a radiographing apparatus, and radiographing conditions such as a registration date, diagnosis information and diagnosis records, and by making these information to be data base, many pieces of information can be preserved together with images. It is possible to conduct CAD for comparing with similar previous case by an image preserving device and conversion to data base, and to download a diagnosis history and a medical treatment history for the similar previous case by correlating them with previous lesions.

Further, on the CAD server, it is possible to administrate usage frequency of CAD software for each facility or for each contract and to charge a fee based on the frequency of usage. AS a method of charging, in addition one based on frequency of usage for CAD software, a method to charge based on the number of times of uploading of images to CAD server, and a method to charge by restricting the types of usable software may be employed. It is also possible to charge depending on whether it is possible to obtain information of diagnosis results or medical treatment records from the similar previous case by utilizing data base information, or not.

Further, if the parameter of CAD used in each facility is preserved in CAD server, it is possible to charge for a fee based on the facility where CAD parameter can be downloaded or on the number of facilities. By making download of CAD parameter to be possible for plural facilities, a parameter of the facility which studies CAD parameter intensively becomes available, which improves CAD efficiency in plural facilities.

By building a system wherein the facility that opens its CAD parameter to the public can receive a fee based on the number of contracts to download that parameter opened to the public by the facility, it is expected that the facility that opened to the public can enjoy a profit, studies of parameter are accelerated, and CAD efficiency is further raised. By downloading also the radiographing conditions simultaneously when downloading the parameter, improvement of CAD efficiency can be expected if the radiographing conditions of the facility that downloaded are made to agree with the radiographing conditions of the facility that has offered the parameter. Further, by applying the parameter of the facility having the radiographing conditions which are extremely different, it is possible to prevent that CAD efficiency is lowered and diagnosis efficiency becomes worse.

### (Operations of the image determining apparatus)

The image determining apparatus is installed in a facility such as a hospital or an image diagnosis center. It is composed of PC (personal computer) such as Windows or Macintosh and of Unix. Further, on the image determining apparatus, an authentication system employing an ID card can be provided. It is further possible to provide a printer that outputs on a sheet of paper or a film and an imager (printer that outputs radiation images). Its software is composed of a browser such as Internet Explorer or Netscape Navigator, or of an exclusive software.

The image determining apparatus is connected, directly or through a network, with an apparatus for obtaining digital images such as FPD (flat panel detector), CR (computed radiography), CT (computed tomography), supersonic waves and laser film digitizer, and it makes it possible to obtain images.

The image determining apparatus is connected, directly or through a network, with an image outputting apparatus such as a printer or an imager, and when images are outputted on a sheet of paper or a film, patient information such as a patient name, patient ID, an age and the distinction of sex, radiographing conditions (tube voltage, mAs value, a type of a filter, a type of a tube and a focus size), and results of detection of CAD are outputted to be a reference for image reading.

The image determining apparatus has an output device for indicating images such as CRT display, and can display the image determined by the image determining apparatus. With respect to the image displayed, patient information such as a patient name, patient ID, an age and the distinction of sex, radiographing conditions (tube voltage, mAs, a type of a filter, a type of a tube and a focus size), and results of detection of CAD are outputted, simultaneously or through switching, to be a reference for image reading.

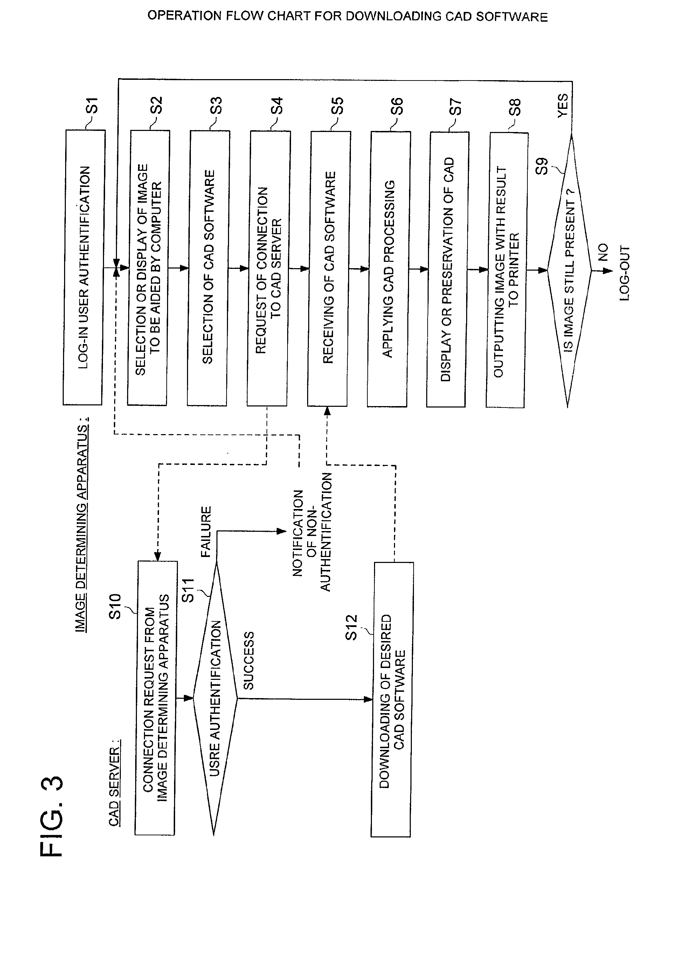

Fig. 3 is an operation flow chart in the case of downloading CAD software. In the drawing, there are shown operations exchanged between image determining apparatus 1 and CAD server 2. On the image determining apparatus 1, there is provided a preserving device (not shown) in which various pieces of information are stored. First, a user logs in for user authentication (S1) User authentication in this case is to check whether or not the user is registered in advance to be entitled to receive the service. User authentication is carried out by sliding ID card through a groove on ID reader 5 (see Fig. 2), for example, and by a password. If it is confirmed that the number which agrees with the ID number thus read is registered (stored) in advance, and when both numbers agree with each other, this means that the authentication is completed. After completion of the user authentication, the user operates a personal computer to select or indicate the image to be subjected to CAD (S2).

When the image to be subjected to CAD is selected, CAD software to be used for that selected CAD is selected (S3). When CAD software is selected, image determining apparatus 1 requests the connection to CAD server 2 (S4).

On the part of the CAD server 2, user authentication for logging in CAD server is conducted (S11) when the request from the image determining apparatus 1 for the connection is received (S10). In this case, the CAD software specified conducts also authentication to check whether the specified CAD software can be downloaded or not. When the user authentication is failed, non-permission notification is sent to the image determining apparatus 1 from the CAD server 2, and the flow goes back to step S2 to conduct processing again. When the user authentication is successful, CAD server 2 conducts downloading of the desired CAD software for the image determining apparatus 1 (S12).

On the part of the image determining apparatus 1, CAD software is received (S5), and the image to be subjected to CAD is subjected to CAD processing (S6). On the part of the image determining apparatus 1, display or preservation of the CAD processing is conducted (S7). The image which has been subjected to CAD processing is stored in the aforementioned preservation device. After completion of the CAD processing, images with results of patient information are outputted to attached printer 6 (see Fig. 2) (S8). On the user side, patient information and images with results of CAD are consulted to be a reference for diagnosis.

Next, in the image determining apparatus 1, it is checked whether the image to be processed still remains or not (S9). When the unprocessed image still remains, the flow goes back to step S2 to continue the processing, while, when no unprocessed image remains, logout is conducted to finish the use of the system.

In the invention, as stated above, administration of CAD algorithm is made to be easy by connecting a plurality of image determining apparatuses of plural facilities representing clients with a computer-aided diagnosis software server (hereinafter referred to as CAD server) through a network. It is further possible to reduce an amount of communication on the network and to shorten a period of time required to obtain results, by downloading the CAD software without sending a large amount of images to CAD server. In general, an amount of image data is large, and application of CAD is smaller in terms of volume.

In the invention, it is possible to call the images and the results of the detection by the computer-aided diagnosis software again and to call the necessary information in case of need to confirm it, because a preservation device for storing various types of information is provided and an output device (printer) for outputting the determined images and information relating to the determined images is provided, both on the image determining apparatus 1.

In this case, information relating to the determined images includes patient information and radiographing conditions. Due to this, patient information, radiographing conditions and results of computer-aided diagnosis software can be outputted on the output device simultaneously together with the images, and images may be correlated with a patient and may be a reference for radiographing, and oversights and errors can be reduced.

Further, in this case, the computer-aided diagnosis system can charge a fee based on the number of times for downloading of the computer-aided diagnosis software and/or types of software that can be downloaded and on the period of time for connection.

In the aforesaid structure, it is possible to control a fee for the single use of CAD software and thereby to create a system which can be used easily even by a user whose frequency of the use is low, by making only the necessary software among plural CAD software to be available, and by charging fees based on the frequency of the use.

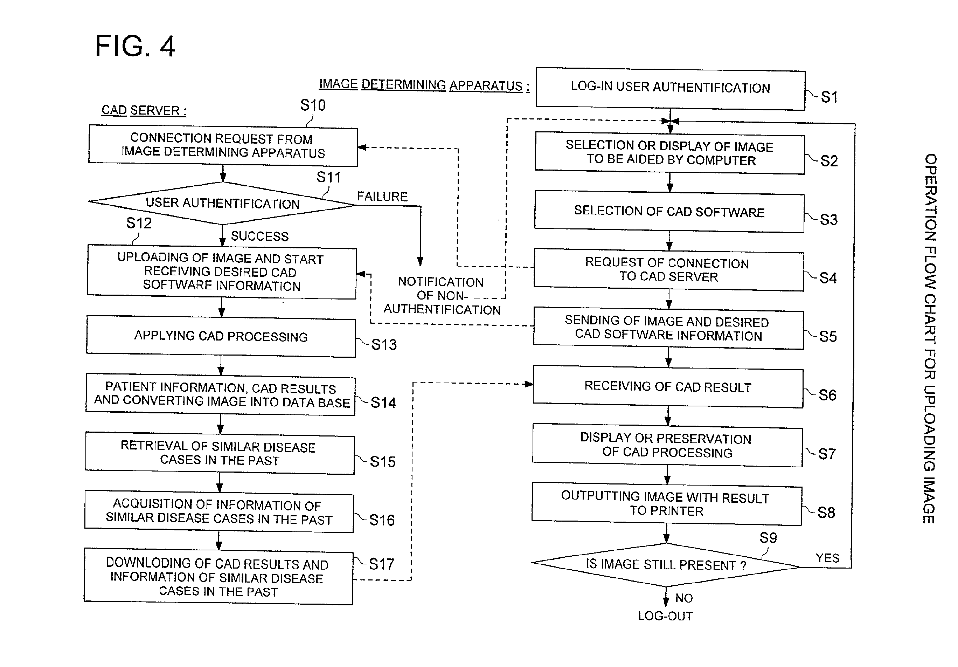

Fig. 4 is an operation flow chart in the case of uploading images. The drawing shows operations exchanged between CAD server 2 and image determining apparatus 1. First, log-in user authentication is conducted (S1). user authentication is carried out by sliding ID card through a groove on ID reader 5 and by a password as stated above. After completion of the authentication, the user selects or indicates the image to be subjected to CAD (S2). Next, an operation section of the image determining apparatus 1 is operated to select the CAD software to be subjected to CAD (S3). After the desired CAD software is selected, the image determining apparatus 1 requests the connection with the CAD server 2 (S4).

On the part of the CAD server 2, the request from the image determining apparatus 1 for the connection is received (S10). Next, CAD server 2 conducts user authentication (S11). In this case, authentication is also conducted to find whether the permission for the use of CAD software is available or not. When the user authentication is failed, non-permission notification is issued, to go back to step S2 to repeat selection of the image to be subjected to CAD processing. When the user authentication is successful, uploading of images and the start receiving of desired CAD software information are conducted (S12). The image determining apparatus 1 transmits (uploads) to CAD server 2 the images and desired CAD software information (S5).

The desired CAD software information includes patient information, radiographing conditions, facility information, information relating to the image determining apparatus and parameter information. This information is used also in the case of preparing data on the CAD server, and facility information as well as information relating to the image determining apparatus are especially important in the present invention wherein images of plural facilities are handled basically at the same time. On the part of the CAD server 2, CAD processing is applied to images received (S13). After completion of the CAD processing, images, patient information, radiographing conditions, CAD results, facility information, information of the image determining apparatus, date of radiographing and date of registration are made to be data base (S14). By registering again the diagnoses records and medical treatment records such as the decided diagnosis result and medical treatment history which have already been registered, data base can be used effectively to improve future diagnosis accuracy. The data base is provided in a storage device (not shown) that is attached to the CAD server 2. Then, the CAD server 2 conducts retrieval of the similar previous cases in case of need (S15). After completion of the retrieval of previous cases, information of the similar previous cases is acquired (S16). The information of the similar previous cases in this case means diagnosis information such as a history of diagnoses and a medical treatment history. After having covered the operations up to this point, the CAD server 2 downloads CAD results and information of the similar previous cases to the image determining apparatus 1 (S17).

On the part of the image determining apparatus 1, CAD results which are sent from the CAD server 2 are received (S6). Then, processing of CAD is displayed on the display section, or it is preserved in the storage device (S7).
Then, images with results are outputted to printer 6 (S8). Next, the image determining apparatus 1 checks whether the image to be processed still remains or not (S9). When the unprocessed image still remains, the flow returns to step S2 to continue the processing. While, when no unprocessed image remains, logout is conducted to finish the processing.

In the invention, as stated above, administration of CAD algorithm on the CAD server is made to be easy by connecting a plurality of image display and preservation device with the CAD server through the network. Further, by transmitting images to CAD server 2, it is possible to administrate the images processed with CAD software and to administrate CAD results intensively, which can be utilized for the application of aging difference processing that compares with previous images.

In the invention, the image determining apparatus 1 has therein a preservation device for preserving images, results of detection conducted by computer-aided diagnosis software and information relating to images and an output device that can output the decided image and information about the decided image as well as the results of detection conducted by the computer-aided software.

In the abovementioned structure, it is possible to call the images and the results of the detection by the computer-aided diagnosis software again and to call the necessary information in case of need to confirm it, because there are provided a preservation device that preserves images and results of detection by computer-aided diagnosis software, information about images and results of computer-aided diagnosis software and an output device for indicating images and preserved information.

In this case, information relating to the determined images includes patient information and radiographing conditions. Due to this, patient information, radiographing conditions and results of computer-aided diagnosis software can be outputted on the output device simultaneously together with the images, and images may be correlated with a patient and may be a reference for radiographing, and oversights and errors can be reduced.

As stated above (step S14 in Fig. 4), CAD server 2 has an image preserving function and/or a data base function, and it can create a data base from either one or some of preservation of image processed by computer-aided diagnosis software and/or patient information, types of opinions, a radiographing date, radiographing conditions, diagnoses results, a medical treatment history, and results of detection by computer-aided diagnosis software.

In the aforesaid structure, it is possible to preserve images and to make results to be a data base, and thereby to administrate intensively image data and patient information, presence or absence of lesions, types of lesions and information of radiographing conditions, thus, calling and retrieval of previous images and similar cases are easy, resulting in easy administration. On the other hand, it is possible to improve computer-aided diagnosis software by utilizing images and information collected intensively, and improvement of accuracy can be expected.

In the aforesaid structure, it is not necessary for the facility to preserve images, and an image preserving system is expected to function. Further, since the previous images are preserved, it is possible to retrieve a previous image from images to be subjected to CAD processing, and to utilize aging difference processing as a computer-aided diagnosis.

Further, in the invention, CAD server 2 has a preserving device for case diagnosis information in which diagnoses results of previous cases and/or medical treatment records are preserved, and has a retrieval device to retrieve similar previous cases from data base information, and thereby, it can download at least one of the diagnoses results of previous cases obtained as a result of retrieval and medical treatment records.

By doing the foregoing, it is possible to retrieve the similar previous cases by retrieving data base information, and to download diagnoses results of previous cases and medical treatment records to image determining apparatus 1, thus, a doctor who reads radiographs is supported and diagnosis efficiency is improved.

In the invention, it is possible to arrange in the computer-aided diagnosis system relating to the invention so that a fee may be charged based on the number to times of uploading of images to be processed by the computer-aided diagnosis software, and/or computer-aided diagnosis software, and/or presence or absence of the use of data base information, and/or types of software processed within the time of connection to CAD server.

In the aforesaid structure, it is possible to charge a fee based on the number of times of uploading of images, or to restrict usable CAD software to charge based on the number of times of the use, and thereby to control a price for single use of CAD software to create a system which can be used easily even by a user whose frequency of use is low.

A system to upload images and a system to download images may be present simultaneously. For example, for the aforesaid purposes of preparation of data base and administration of images, images and information about the images are uploaded first to prepare the data base and to conduct CAD processing on the CAD server, and the results are downloaded. When changing a parameter to conduct processing with the same CAD software twice or more or when processing another CAD software for the same image, the desired CAD software may be downloaded for processing for the purpose of reducing an amount of communication on the network to obtain the results more rapidly even slightly, because preparation of basic data base has already been completed on the CAD server.

### EFFECTS OF THE INVENTION

As stated above, the invention makes it possible to provide a computer-aided diagnosis system wherein each apparatus is connected to others through a network so that efficiency thereof for use can be improved.

## Claims

1. A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed, downloads the computer-aided diagnosis software from the software server, and processes the radiographic image data by utilizing the downloaded computer-aided diagnosis software.

2. The computer-aided diagnosis system of claim 1, wherein the software server has a plurality of different kinds of computer-aided diagnosis softwares from each other, and the image determining apparatus selects the computer-aided diagnosis software to process the radiographic image data and uploads information of the result of selection to the software server.

3. The computer-aided diagnosis system of claim 1, wherein the image determining apparatus comprises:
a storing device capable of storing at least one of the radiographic image data, processed result data of processing the radiographic image data by the computer-aided diagnosis software, and information relating to the radiographic image data; and
an output device capable of outputting at least one of the radiographic image data, the processed result data and the information relating to the radiographic image data.

4. The computer-aided diagnosis system of claim 3, wherein the information relating to the radiographic image data is at least one of a patient information and an exposure condition of the radiographic image data.

5. The computer-aided diagnosis system of claim 1, wherein the software server charges by at least one of the number of times for the image determining apparatus to download the computer-aided diagnosis software, the kind of downloaded computer-aided diagnosis software and the connecting time from the image determining apparatus to the software server.

6. The computer-aided diagnosis system of claim 1, wherein the image determining apparatus further comprises a parameter changing device to change the parameter of the computer-aided diagnosis software downloaded from the software server.

7. The computer-aided diagnosis system of claim 1, wherein a plurality of the image determining apparatus connects to the software server.

8. A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed and uploads the determined radiographic image data to the software server, the software server processes the uploaded radiographic image data by utilizing the computer-aided diagnosis software, and the image determining apparatus downloads the processed result data of processing the radiographic image data by the computer-aided diagnosis software from the software server.

9. The computer-aided diagnosis system of claim 8, wherein the software server has a plurality of different kinds of computer-aided diagnosis softwares from each other, and the image determining apparatus selects the computer-aided diagnosis software to process the radiographic image data and uploads information of the result of selection to the software server.

10. The computer-aided diagnosis system of claim 8,
wherein the image determining apparatus comprises:
a storing apparatus capable of storing at least one of the radiographic image data, processed result data of processing the radiographic image data by the computer-aided diagnosis software, and information relating to the radiographic image data; and
an output apparatus capable of outputting at least one of the radiographic image data, the processed result data and the information relating to the radiographic image data.

11. The computer-aided diagnosis system of claim 10, wherein the information relating to the radiographic image data is at least one of a patient information and an exposure condition of the radiographic image data.

12. The computer-aided diagnosis system of claim 8, wherein the image determining apparatus uploads information relating to the radiographic image data, which is uploaded to the software server, to the software server.

13. The computer-aided diagnosis system of claim 12, wherein the information relating to the radiographic image data is at least one of patient information, information of exposure condition of the radiographic image data and information relating to the image determining apparatus.

14. The computer-aided diagnosis system of claim 13, wherein the information relating to the image determining apparatus is information of a facility having the image determining apparatus.

15. The computer-aided diagnosis system of claim 12, wherein the software server comprises:
an image storing device capable of storing the uploaded radiographic image data; and
a database making device capable of making a database of at least one of processed result data of processing the radiographic image data by the computer-aided diagnosis software and the information relating to the radiographic image data.

16. The computer-aided diagnosis system of claim 15, wherein the information relating to the radiographic image data is information of facility having the image determining apparatus.

17. The computer-aided diagnosis system of claim 8, wherein the software server comprises:
a previous-case diagnosis data storing device capable of making a database of at least one of data of a diagnosing result of a previous case and data of a previous treatment and storing the data; and
a retrieving device capable of retrieving the data in the storing device,
wherein the image determining apparatus retrieves the data in the storing device by utilizing the retrieving device and downloads at least one of obtained data as the result of retrieving.

18. The computer-aided diagnosis system of claim 15, wherein software server further comprises a subtraction processing device capable of retrieving a previous data of uploaded radiographic image data in the image storing device and subjects the previous data and the uploaded radiographic image data to a temporal subtraction processing, and the image determining apparatus download the result of the temporal subtraction processing.

19. The computer-aided diagnosis system of claim 8, wherein the software server charges by at least one of the number of times for the image determining apparatus to upload the radiographic image data to the software server, the number of times of processing the uploaded radiographic image data by the computer-aided diagnosis software, and the connecting time from the image determining apparatus to the software server.

20. The computer-aided diagnosis system of claim 8, wherein the image determining apparatus further comprises a parameter data making device to make parameter data capable of changing a parameter of the computer-aided diagnosis software in the software server, and the image determining apparatus uploads the parameter data made by the parameter data making device with the radiographic image data to the software server, and the software server applies the uploaded parameter data to the computer-aided diagnosis software.

21. The computer-aided diagnosis system of claim 20, wherein a plurality of image determining apparatuses, which belong to different facilities from each other, connect to the software server, and the software server stores the uploaded parameter data depending on the facilities, and the plurality of image determining apparatuses download the uploaded parameter data.

22. The computer-aided diagnosis system of claim 21, wherein the image determining apparatus uploads information of exposure condition of the radiographic image data to the software server, the software server makes a database of the information of exposure condition of the radiographic image data, and the image determining apparatus downloads the information of exposure condition of the radiographic image data with the parameter data from the software server.

23. The computer-aided diagnosis system of claim 21, wherein the software server charges by at least one of the facility downloading the parameter data and the number of the facilities downloading the parameter data.

24. The computer-aided diagnosis system of claim 8, wherein a plurality of image determining apparatus connect the software server.

25. A computer-aided diagnosis system comprising:
a computer-aided diagnosis software server having a computer-aided diagnosis software capable of processing a radiographic image data to detecting an abnormality; and
an image determining apparatus interconnected with the software server via a network,
wherein the image determining apparatus determines the radiographic image data to be processed, and
when the image determining apparatus downloads the computer-aided diagnosis software from the software server, the image determining apparatus processes the radiographic image data by utilizing the downloaded computer-aided diagnosis software, and
when the image determining apparatus uploads the determined radiographic image data to the software server, the software server processes the uploaded radiographic image data by utilizing the computer-aided diagnosis software, and the image determining apparatus downloads the processed result data of processing the radiographic image data by the computer-aided diagnosis software from the software server.
